# EUROPÄISCHE PATENTSCHRIFT

(11) **EP 1 243 258 B1**
(45) Veröffentlichungstag und Bekanntmachung des Hinweises auf die Patenterteilung: **18.02.2004**
(21) Anmeldenummer: 02005024.1
(22) Anmeldetag: 06.03.2002
(51) Int. Cl.: A61K 9/22, A61K 31/7036, A61L 31/16, A61L 27/54

(54) **Herstellung und Verwendung einer Antibiotikum-/Antibiotika-Zubereitung**
Production of a preparation comprising one or more antibiotics
Production et utilisation d'une préparation comprenant un ou plusieurs antibiotiques

(30) Priorität: 22.03.2001 DE 10114245
(43) Veröffentlichungstag der Anmeldung: 25.09.2002
(73) Patentinhaber: Heraeus Kulzer GmbH & Co.KG, 63450 Hanau (DE)
(72) Erfinder: Vogt, Sebastian F., Dr., 07749 Jena (DE); Schnabelrauch, Matthias, Dr., 07745 Jena (DE); Kühn, Klaus-Dieter, Dr., 35041 Marburg (DE)
(74) Vertreter: Kühn, Hans-Christian

(56) Entgegenhaltungen:
- FR-A- 2 668 367
- GB-A- 1 478 240
- US-A- 4 291 013
- US-A- 5 670 142
- DATABASE WPI Derwent Publications Ltd., London, GB; AN 1995-373533 XP002200970 BELOV EV ET AL: "Haemostatic and antibacterial sponges for surgical use" & WO 95 27517 A (LYZION AUSTRALIA PTY LTD;ROSSIISKY NII GEMATOL (RU)), 19. Oktober 1995 (1995-10-19)

## Beschreibung

Die vorliegende Erfindung betrifft die Herstellung und Verwendung einer Antibiotikum/Antibiotika-Zubereitung mit verzögerter Wirkstofffreisetzung für die Human- und Veterinärmedizin zur Behandlung lokaler mikrobieller Infektionen im Hart- und im Weichgewebe.

Die Behandlung lokaler mikrobieller Infektionen des Weich- und Hartgewebes in der Human- und Veterinärmedizin erfordert hohe lokale Antibiotika-Konzentrationen im infizierten Gewebebereich. Es ist seit langem bekannt, daß eine systemische Anwendung von Antibiotika mit einer Reihe von Problemen behaftet ist. Bei der systemischen Anwendung ist es oft erforderlich sehr hohe Antibiotika-Dosen einzusetzen, damit im infizierten Gewebe antimikrobiell wirksame Antibiotika-Konzentrationen erzielt werden. Dadurch kann es insbesondere bei den Aminoglykosid-Antibiotika und bei den Antibiotika vom Tetracyclin-Typ auf Grund ihrer Nephro- und Ototoxizität zu schwerwiegenden Schädigungen des Organismus kommen. Daher lag der Gedanke nahe, Antibiotika in lokal applizierbaren Freisetzungssystemen einzusetzen, bzw. sie in geeignete Depotformen zu überführen.

Depotsysteme zur verzögerten Freisetzung von Antibiotika für die Behandlung von lokalen Infektionen sind Gegenstand einer Vielzahl von Veröffentlichungen und Patenten. Diese kann man allgemein nach zwei grundlegenden Retardierungsmechanismen einteilen. Das eine Wirkungsprinzip besteht in der physikalischen Fixierung der Antibiotika durch Adsorption an eine Matrix bzw. durch Einschluß in eine nichtresorbierbare oder resorbierbare Matrix. Das zweite, chemische Verzögerungsprinzip besteht in der Verwendung von schwerlöslichen Antibiotika-Salzen, die sich nach entsprechender Applikation im humanen oder tierischen Organismus langsam unter Wirkstofffreisetzung auflösen.

Die physikalische Fixierung von Antibiotika unter Verwendung von nichtresorbierbaren Kunststoffen war Inhalt einer Reihe von Patenten, von denen hier nur einige exemplarisch aufgeführt werden. So schlägt Klemm (K. Klemm: Surgical synthetic-resin material and method of treating osteomyelitis. 13.05.1975, **US 3,882,858**) die Behandlung von Osteomyelitis mit Kunststoffpartikeln aus Polymethacrylat, Polyacrylat sowie deren Kopolymeren vor, die mit Gentamicin oder anderen Antibiotika beladen sind. Klemm beschreibt die Anwendung von Septopal (K. Klemm: Septopal - a new way of local antibiotic therapy. In T. J. G. Van Rens, F. H. Kayser (Eds.), Local antibiotic Treatment in Osteomyelitis and Soft-Tissue Infections, Excerpta Medica, Amsterdam (1981) 24-31; K. Klemm: Antibiotic beat chains. Clin. Orthop. Relat. Res. 295 (1993) 63-76.). Hierbei handelt es sich um kommerziell verfügbare Gentamicin-freisetzende Ketten aus Polymethacrylat. Heuser und Dingeldein beschreiben eine Komposition auf der Basis von Antibiotika und Polymethymethacrylat bzw. Polyacrylat, der als zusätzliche Komponente Aminosäuren zugefügt sind (D. Heuser, E. Dingeldein: Synthetic resin-base, antibiotic compositions containing amino acids. 04.04.1980, **US 4,191,740**; D. Heuser, E. Dingeldein: Synthetic resin-base, antibiotic compositions containing amino acids. 11.11.1980, **US 4,233,287**). Weiterhin wurden auch Antibiotika, insbesondere Aminoglykosid-Antibiotika, in Knochenzemente integriert (A. Gross, R. Schaefer, S. Reiss: Bone cement compositions containing gentamicin. 22.11.1977, **US 4,059,684**; A. Welch: Antibiotics in acrylic bone cement. In vitro studies. J. Biomed. Mater. Res. 12 (1978) 679; R. A. Elson, A. E. Jephott, D. B. McGechie, D. Vereitas: Antiobiotic-loaded acrylic cement. J. Bone Joint Surg. 59B (1977) 200-205.

Die physikalische Fixierung von Antibiotika mit Hilfe von resorbierbaren Kunststoffen, insbesondere von Polyestern der α-Hydroxycarbonsäuren, war ebenfalls Gegenstand einer Reihe von Publikationen, von denen hier ebenfalls nur einige exemplarisch referiert werden. Sampath et. al. schlagen ein Gentamicin-freisetzendes System bestehend aus Poly-L-lactid und Gentamicin vor, das durch Verpressung von Poly-L-lactid/Gentamicin-Mikrokapseln hergestellt wurde (S. S. Sampath, K. Garvin, D. H. Robinson: Preparation and characterization of biodegradable poly(-L-lactic acid) gentamicin delivery systems. Int. J. Pharmaceutics 78 (1992) 165-174). Dieses System zeigt in Abhängigkeit von der eingesetzten Menge an Gentamicin eine nicht unbeträchtliche Verzögerung der Wirkstofffreisetzung. Bei einem ähnlichen System wurde Poly-D,L-lactid zur Herstellung von wirkstoffenthaltenden Mikrosphären genutzt (R. Bodmeier, J. W. McGinity: The preparation and evaluation of drug-containing poly(D,L-lactide) microspheres formed by solvent evaporation method. Pharm. Res. 4 (1987) 465-471). Von Fries und Schlapp werden ebenfalls Mikropartikel aus Polylactid beschrieben, die mit Kollagen/Gentamicinsulfat beschichtet sind ( W. Friess, M. Schlapp: Advanced implants for local delivery of gentamicin. Sixth World Biomaterials Congress Transactions (2000) 1488). Diese beschichteten Mikrosphären zeigten nur eine sehr geringe Tendenz, die Gentamicin-Freisetzung zu verzögern. Von Schmidt et al. wurden gentamicinenthaltende resorbierbare Formkörper vorgeschlagen (C. Schmidt, R. Wenz, B. Nies, F. Moll: Antibiotic in vivo/in vitro release, histocompatibility and biodegradation of gentamicin implants based on lactic acid polymers and copolymers. J. Control. Release 37 (1995) 83-94). Diese Körper wurden durch Verpressung von Gemischen aus Gentamicin-Sulfat/Poly-L-lactid, Gentamicin-Sulfat/Poly-D,L-lactid und Gentamicinsulfat/Poly-D,L-lactid-co-glykolid hergestellt. Diese Depotpräparate setzten zirka neunzig Prozent des Antibiotikums innerhalb von vierundzwanzig Stunden frei.

Neben der physikalischen Fixierung von Antibiotika unter Verwendung von Kunststoffen wurden auch zahlreiche anorganische Systeme mit retardierender Wirkung beschrieben. Im folgenden werden exemplarisch nur einige der mit Calciumsulfat hergestellten Systeme kurz referiert. So wird von Randolph et al. ein retardierendes System beschrieben, daß auf dem Einschluß von Wirkstoffen in eine Calciumsulfat-Matrix beruht (D. A. Randolph, J. L. Negri, T. R. Devine, S. Gitelis: Calcium sulfate controlled release matrix. 15.09.1998, **US 5,807,567**). Die Herstellung dieser Calciumsulfat-Pellets erfolgt dabei ausgehend von einem Gemisch aus α-Calciumsulfat-Hemihydrat, β-Calciumsulfat-Hemihydrat, einem Additiv und Wasser. Die Härtung erfolgt durch Bildung von Calciumsulfat-Dihydrat. Turner et al. beschreiben Tabletten aus Calciumsulfat, die Tobramycin enthalten und zur Behandlung von Medullar-Defekten Verwendung finden sollen (T. M. Turner, R. M. Urban, S. Gitelis, A. M. Lawrence-Smith, D. J. Hall: Delivery of tobramycin using calcium sulfate tablets to graft a large medullary defect: Local and systemic effects. Sixth World Biomaterials Congress Transactions (2000) 767.) Ähnliche Freisetzungssysteme aus Calciumsulfat, aber mit Amikacin-Sulfat, werden ebenfalls beschrieben (D. W. Petersen, W.O. Haggard, L. H. Morris, K. C. Richelsoph, J. E. Parr: Elution of amikacin from calcium sulfate pellets: An in vitro study. Sixth World Biomaterials Congress Transactions (2000) 767).

Bisher fanden schwerlösliche Salze der Aminoglykosid-Antibiotika und der Lincosamid-Antibiotika relativ geringe Beachtung für die Herstellung von Depotpräparaten. Die Bildung von schwerlöslichen Salzen bzw. Chelaten der Antibiotika des Tetracyclintyps ist seit Jahrzehnten allgemeiner Kenntnisstand. So beschreibt Folch Vazquez die Herstellung von Tetracyclindodecylsulfat durch Umsetzung von Tetracyclinhydrochlorid mit Natriumdodecylsulfat in Wasser (C. Folch Vazquez: Tetracycline lauryl sulfate. 08.02.1966, **ES 3 309 402**; C. Folch Vazquez: Tetracycline derivatives. 09.01.1967, **NL 6609490**) Alternativ kann die Herstellung auch ausgehend von Tetracyclin und der Dodecylschwefelsäure erfolgen ( C. Folch Vazquez: Tetracycline lauryl sulfate. 08.02.1966, **ES 322 771**). Weiterhin wurde auch die Verwendung von Tetracylin-Sulfamaten zur antibiotischen Therapie vorgeschlagen (A. Jurando, J. M. Puigmarti: Antibiotic tetracycline sulfamate and its derivatives. 27.10.1970, **US 3,536,759**; Anonym: Antibiotic tetracycline alkylsulfamates. 16.10.1969, **ES 354 173**; C. Ciuro, A. Jurado: Stability of a tetracycline derivative. Afinidad 28 (292) 1971, 1333-5.). Bei den Aminoglykosid-Antibiotika sind ebenfalls eine Reihe von schwerlöslichen Salzen prinzipiell bekannt. So wurde beim Gentamicin die Darstellung schwerlöslicher Salze basierend auf höheren Fettsäuren, Arylalkylcarbonsäuren, Alkylsulfaten und Alkylsulfonaten beschrieben ( G. M. Luedemann, M. J. Weinstein: Gentamycin and method of production. 16.07.1962, **US 3,091,572** ). Exemplarisch sind dafür Gentamicin-Salze der Laurinsäure, der Stearinsäure, der Palmitinsäure, der Ölsäure, der Phenylbuttersäure, der Naphthalen-1-carbonsäure, der Laurylschwefelsäure und der Dodecylbenzensulfonsäure. Diese Salze erwiesen sich vielfach als unvorteilhaft, weil sie wachsartige, hydrophobe Substanzen darstellen, die eine galenische Verwendung behindern. Trotzdem wurden Festtsäuresalze von Gentamicin und von Etamycin aus der freien Base bzw. aus ihren Salzen in Wasser bei 50-80°C synthetisiert (H. Voege, P. Stadler, H. J. Zeiler, S. Samaan, K. G. Metzger: Sparinglysoluble salts of aminoglycosides and formations containing them with inhibited substancerelease. 28.12.1982 **DE 3 248 328**). Diese Antibiotika-Fettsäuresalze sollen als Injektionspräparate geeignet sein. Die Herstellung von Gentamicindodecylsulfat und dessen Verwendung in Salben, Cremes wurde ebenfalls beschrieben (C. Folch Vazquez: Gentamicin derivates. 29.10.1974, **BE 821 600**). Eine neuere Entwicklung stellen schwerlösliche Aminoglykosid-Flavonoid-Phosphate dar (H. Wahlig, E. Dingeldein, R. Kirchlechner, D. Orth, W. Rogalski: Flavonoid phosphate salts of aminoglycoside antibiotics. 13.10.1986, **US 4,617,293**). Es werden die Salze der Phosphorsäurehalbester von Derivaten der Hydroxyflavane, Hydroxyflavene, Hydroxyflavanone, Hydroxyflavone und Hydroxyflavylium beschrieben. Besonders bevorzugt sind die Derivate der Flavanone und der Flavone. Diese schwerlöslichen Salze sollen als Depotpräparate Verwendung finden. So werden zum Beispiel diese Salze in Kollagenvliese eingebracht ( H. Wahlig, E. Dingeldein, D. Braun: Medicinally useful, shaped mass of collagen resorbable in the body. 22.09.1981, **US 4,291,013**). Weiterhin wurden auch künstliche Herzklappen mit diesen schwerlöslichen Gentamicin-Salzen, Gentamicin-Crobefat, imprägniert (M. Cimbollek, B.Nies, R. Wenz, J. Kreuter: Antibiotic-impregnated heart valve sewing rings for treatment and prophylaxis of bacterial endocarditis. Antimicrob. Agents Chemother. 40(6) (1996)1432-1437). Interessant ist bei diesem Patent insbesondere, daß ein Gemisch aus leichtlöslichem Gentamicin-Sulfat und schwerlöslichem Gentamicin-Crobefat eingesetzt wird. Das Ziel war dabei, daß einerseits nach Einbringung der Herzklappenringe in den Organismus bzw. in eine Modellflüssigkeit eine hohe initiale Gentamicin-Konzentration durch das leichtlösliche Gentamicin-Sulfat erreicht wird und das andererseits durch das relativ schwerlösliche Gentamicin-Crobefat eine Freisetzung von Gentamicin über einen längeren Zeitraum möglich wird. Das bedeutet, die zeitabhängige Freisetzung des Gentamicins wird durch das Verhältnis aus leichtlöslichem Gentamicin-Sulfat und schwerlöslichem Gentamicin-Crobefat gesteuert. Für eine gezielte Einstellung des Freisetzungsverhaltens ist es daher erforderlich, die beiden Gentamicin-Salze in definierten Mengenverhältnissen in die galenischen Formulierungen einzubringen. Diese Methode der Depotbildung durch Kombination eines leichtlöslichen Antibiotika-Salzes mit einem schwerlöslichen Antibiotika-Salz setzt die Verfügbarkeit einer reinen schwerlöslichem Salzform eines Antibiotikums voraus.

Zusammenfassend kann festgestellt werden, daß die bekannten Antibiotika-Depotsysteme mit physikalisch verursachter Verzögerung der Antibiotika-Freisetzung in starkem Maße von der Zusammensetzung und Struktur der verwendeten Matrix abhängen. Weiterhin ist der Herstellungsprozeß dieser Antibiotika-Systeme von nicht unerheblichem Einfluß auf das Freisetzungsverhalten. Die Verwendung von schwerlöslichen Antibiotika-Salzen erscheint prinzipiell die Möglichkeit zu eröffnen, weitgehend Matrix-unabhängige retardierend wirkende Systeme zu erzeugen, wie das Patent **US 4,617,293** zeigt. Der bisherige Nachteil dieser Systeme besteht darin, daß für jedes verwendete Antibiotikum aus den Gruppen der Aminoglykosid-Antibiotika, der Lincosamid-Antibiotika und Tetracyclin-Antibiotika eine spezielle Salzform vor der Herstellung der Depotpräparate synthetisiert werden muß.

Der vorliegenden Erfindung liegt die Aufgabe zugrunde, ein Verfahren zur Herstellung einer Antibiotikum-/Antibiotika-Zubereitung mit verzögernder Wirkstofffreisetzung zur Behandlung lokaler mikrobieller Infektionen im Knochen- und im Weichgewebe für die Human- und Veterinärmedizin bereitzustellen, das die Nachteile der bekannten retardierenden Antibiotika-Formulierungen überwindet. Angestrebt wird eine Antibiotikum-/Antibiotika-Zubereitung, die eine kontrollierte Antibiotika-Freisetzung über einen Zeitraum bis ungefähr drei Wochen ermöglicht. Der Mechanismus der verzögerten Wirkstofffreisetzung sollte weitgehend unabhängig von Trägermaterialien sein und nicht auf Adsorptionseffekten an den Oberflächen von Trägermaterialien beruhen. Angestrebt wird eine Antibiotikum-/Antibiotika-Zubereitung, die unter Beibehaltung der Wirkstoffretardierung sowohl mit resorbierbaren als auch nichtresorbierbaren Hilfsstoffen unterschiedlicher Struktur zu Implantaten verarbeitet werden kann. Weiterhin soll die Art und Weise der Antibiotikum-/Antibiotika-Zubereitung nicht nur für ein spezielles Antibiotikum anwendbar sein, sondern es sollte vielmehr für eine Reihe Antibiotika ähnlicher Struktur geeignet sein.

Der Erfindung liegt der überraschende Befund zu Grunde, daß ein Gemisch bestehend aus Wasser, einer amphiphilen Komponente eines Vertreters der Alkylsulfate, Arylsulfate, Alkylarylsulfate, Cycloalkylsulfate, Alkylcycloalkylsulfate, Alkylsulfamate, Cycloalkylsulfamate, Alkylcycloalkylsulfamate, Arylsulfamate, Alkylarylsulfamate, Alkylsulfonate, Fettsäure-2-sulfonate, Arylsulfonate, Alkylarylsulfonate, Cycloalkylsulfonate, Alkylcycloalkylsulfonate, Alkyldisulfate, Cycloalkyldisulfate, Alkyldisulfonate, Cycloalkyldisulfonate, Aryldisulfonate, Alkylaryldisulfonate, Aryltrisulfonate und Alkylaryltrisulfonate sowie mindestens eine antibiotische Komponente aus der Gruppe der Aminoglykosid-Antibiotika, der Lincosamid-Antibiotika und Tetracyclin-Antibiotika, **einer** anorganischen Hilfskomponente **ggf.** einer organischen **Hilfskomponente,** und ggf. mindestens einer biologisch aktiven Hilfskomponente - **wobei** Calciumhydrogenphosphat, Calciumhydrogenphosphat-Dihydrat, Hydroxylapatit, Fluorapatit, Calciumpolyphosphat, Tricalciumphosphat, Tetracalciumphosphat, Calciumsulfat, Calciumsulfat-Hemihydrat, Calciumsulfat-Dihydrat, Calciumlactat, Natriumhydrogencarbonat, Calciumcarbonat, Magnesiumcarbonat, Calciumhydroxid, Magnesiumhydroxid, Magnesiumoxid **oder** Gemische dieser Stoffe in Form grobdisperser (0,5 bis 2 mm) und/oder hochdisperser Pulver; oder resorbierbare Gläser, nichtresorbierbare Gläser, resorbierbare Glaskeramik, nichtresorbierbare Glaskeramik, resorbierbare Keramik und nichtresorbierbare Keramik als anorganische Hilfskomponente verwendet werden - eine Wirkstoff-Formulierung ergibt, die zu Formkörpern und/oder Granulaten und/oder Pulvern und/oder Folien und/oder Vliesen und/oder Fäden vorzugsweise mittels Pressen und/oder Strangpressen und/oder Mahlen und/oder Kalandrieren und/oder Gießen und/oder Spinnen und/oder Sintern verarbeitet werden kann. Überraschend ist, daß diese Formkörper und Beschichtungen nach Einbringung in ein wässriges Milieu eine verzögerte Antibiotika-Freisetzung über einen Zeitraum von mehreren Tagen bis zu mehreren Wochen zeigen.

Die nachfolgenden vorteilhaften Ausgestaltungen haben sich in der Praxis bewährt.

Erfindungsgemäß ist es vorteilhaft, daß die amphiphile Komponente aus der Gruppe der Alkylsulfate, Arylsulfate, Alkylarylsulfate, Cycloalkylsulfate und Alkylcycloalkylsulfate als Halbester in der Natriumsalzform und/oder in der Kaliumsalzform und/oder in der Ammoniumsalzform und/oder in der Trialkylammoniumsalzform und/oder in der Dialkylammoniumsalzform und/oder der Monoalkylammoniumsalzform und/oder in der Triarylammoniumsalzform und/oder in der Diarylammoniumsalzform und/oder in der Arylammoniumsalzform und/oder in der Alkyldiarylammoniumsalzform und/oder in der Dialkylarylammoniumsalzform und/oder der Tricycloalkylammoniumsalzform und/oder der Dicycloalkylammoniumsalzform und/oder der Monocycloalkylammoniumsalzform und/oder der Alkyldicycloalkylammoniumsalzform und/oder in der Dialkylcycloalkylammoniumsalzform und/oder in der Säureform und/oder in der Anhydridform vorliegt.

Weiterhin ist erfindungsgemäß vorteilhaft, daß die amphiphile Komponente aus der Gruppe der Alkylsulfonate, Fettsäure-2-sulfonate, Alkylsulfamate, Cycloalkylsulfamate, Arylsulfamate, Alkylarylsulfamate, Arylsulfonate, Alkylarylsulfonate, Cycloaalkylsulfonate, Alkylcycloalkylsulfonate, Alkyldisulfate, Cycloalkyldisulfate, Alkyldisulfonate, Cycloalkyldisulfonate, Aryldisulfonate, Alkylaryldisulfonate, Aryltrisulfonate und Alkylaryltrisulfonate in der Natriumsalzform und/oder in der Kaliumsalzform und/oder in der Ammoniumsalzform und/oder in der Trialkylammoniumsalzform und/oder in der Dialkylammoniumsalzform und/oder der Monoalkylammoniumsalzform und/oder in der Triarylammoniumsalzform und/oder in der Diarylammoniumsalzform und/oder in der Arylammoniumsalzform und/oder in der Alkyldiarylammoniumsalzform und/oder in der Dialkylarylammoniumsalzform und/oder der Tricycloalkylammoniumsalzform und/oder der Dicycloalkylammoniumsalzform und/oder der Monocycloalkylammoniumsalzform und/oder der Alkyldicycloalkylammoniumsalzform und/oder in der Dialkylcycloalkylammoniumsalzform und/oder in der Sulfonsäureform und/oder in der Sulfonsäureanhydridform vorliegt.

Erfindungsgemäß vorteilhaft ist auch, daß die antibiotische Komponente mindestens eine Aminogruppe enthält.

Ferner ist es erfindungsgemäß vorteilhaft, daß als amphiphile Komponente Alkylsulfate, Cycloalkylsulfate, Cycloalkylalkylsulfate, Arytsulfate, Alkylarylsulfate, Alkylsulfamate, Cycloalkylsulfamate, Alkylcycloalkylsulfamate, Arylsulfamate, Alkylarylsulfamate, Alkylsulfonate, Fettsäure-2-sulfonate, Cycloalkylsulfonate, Cycloalkylalkylsulfonate, Arylsulfonate, und Alkylarylsulfonate mit jeweils 6 bis 30 Kohlenstoffatomen bevorzugt sind.

Erfindungsgemäß vorteilhaft ist, daß aus monocyclischen, dicyclischen, tricyclischen, tetracydischen, pentacyclischen, hexacyclischen, heptacyclischen und octacyclischen aromatischen Ringsystemen aufgebaute Arylsulfate, Alkylarylsulfate, Arylsulfamate, Alkylarylsulfamate, Aryldisulfonate, Alkylaryldisulfonate, Aryltrisulfonate und Alkylaryltrisulfonate als amphiphile Komponente bevorzugt sind.

Erfindungsgemäß vorteilhaft ist, daß aus monocyclischen, dicyclischen, tricyclischen, tetracyclischen, pentacyclischen, hexacydischen, heptacyclischen und octacyclischen gesättigten Ring-systemen aufgebaute Cycloalkylsulfate, Alkylcycloalkylsulfate, Cycloalkylsulfonate, Alkylcycloalkylsulfonate, Cycloalkylsulfamate, und Alkylcycloalkylsulfamate als amphiphile Komponente bevorzugt sind.

Erfindungsgemäß vorteilhaft ist, daß Natriumdodecylsulfat, Natriumtetradecylsulfat, Natriumhexadecylsulfat, Natriumoctadecylsulfat, Natriumdocosanylsulfat, Natriumdodecylsulfonat, Natriumtetradecylsulfonat, Natriumhexadecylsulfonat, Natriumoctadecylsulfonat, Natriumdodecylbenzylsulfonat und Natriumcholesterolsulfat als amphiphile Komponente besonders bevorzugt werden.

Weiterhin ist es erfindungsgemäß von Vorteil, daß als antibiotische Komponente Allomycin, Amicetin, Amikacin, Apramycin, Bekanamycin, Betamicin, Butirosin, Destomycin, Dibekacin, Dihydrostreptomycin, Flambamycin, Fortimycin A, Fortimycin B, Framycetin, Gentamicin, Hikizimycin, Homomycin, Hybrimycin, Hygromycin B, Kanamycin, Kasuhamycin, Lividomycin, Minosaminoycin, Neomycin, Netilmicin, Paromomycin, Parvulomycin, Puromycin A, Ribostamycin, Rimocidin, Ristosamin, Ristomycin, Sagamycin, Sisomicin, Sorbistin, Spectinomycin, Streptomycin, Tobramycin, Tunicamycin, Verdamycin aus der Gruppe der Aminoglykosid-Antibiotika bevorzugt werden.

Erfindungsgemäß vorteilhaft ist, daß als antibiotische Komponente Clindamycin und Lincomycin aus der Gruppe der Lincosamid-Antibiotika bevorzugt werden.

Erfindungsgemäß vorteilhaft ist, daß als antibiotische Komponente Tetracyclin, Chlortetracyclin, Oxytetracylin, Demethylchlortetracyclin, Methacyclin, Doxycyclin, Rolitetracyclin und Minocyclin aus der Gruppe der Tetracyclin-Antibiotika bevorzugt werden.

Es ist auch erfindungsgemäß von Vorteil, daß die antibiotische Komponente in der protonierten Salzform vorliegt, wobei Chlorid-Ionen, Bromid-Ionen, Hydrogensulfat-Ionen, Sulfat-Ionen, Dihydrogenphosphat-Ionen, Hydrogenphosphat-Ionen, Phosphat-Ionen, Acetat-Ionen, Succinat-Ionen und Lactat-Ionen als Gegenionen bevorzugt sind.

Erfindungsgemäß ist es weiterhin von Vorteil, daß bevorzugt 0,01 bis 10 Stoffmengenteile der amphiphilen Komponente mit einem Stoffmengenteil der antibiotischen Komponente vermischt sind.

Erfindungsgemäß ist es vorteilhaft, daß durch das Verhältnis der Stoffmenge der amphiphilen Komponente zur Stoffmenge der antibiotischen Komponente der Anteil der verzögert freigesetzten antibiotischen Komponente an der Gesamtmenge der antibiotischen Komponente bestimmt wird.

Erfindungsgemäß ist es vorteilhaft, daß die wasserfreie, organische Hilfskomponente hydrolytisch spaltbare Carbonsäureesterbindungen und/oder hydrolytisch spaltbare Carbonsäureamidbindungen und/oder hydrolytisch spaltbare Carbonsäureanhydridbindungen und/oder hydrolytisch spaltbare Phosphorsäuresterbindungen und/oder hydrolytisch spaltbare Phosphorsäureamidbindungen und/oder enzymatisch spaltbare Carbonsäureesterbindungen und/oder enzymatisch spaltbare Carbonsäureamidbindungen und/oder enzymatisch spaltbare Carbonsäureanhydridbindungen und/oder enzymatisch spaltbare Phosphorsäuresterbindungen und/oder enzymatisch spaltbare Phosphorsäureamidbindungen aufweist.

Es ist auch erfindungsgemäß von Vorteil, daß Oligoester und Polyester der L-Milchsäure und/oder der D-Milchsäure und/oder der 2-Hydroxyethansäure und/oder der 2-Hydroxyethoxyethansäure und/oder der 3-Hydroxybutansäure und/oder der 4-Hydroxybutansäure und/oder der 4-Hydroxyhexansäure und/oder der 6-Hydroxyhexansäure und ggf. Kooligoester und/oder Kopolyester und ggf. Teroligoester und/oder Terpolyester dieser Hydroxycarbonsäuren als wasserfreie, organische Hilfskomponente verwendet werden.

Es ist erfindungsgemäß vorteilhaft, daß Oligoamide und/oder Polyamide als wasserfreie, organische Hilfskomponente verwendet werden, die als Bestandteile Aminosäuren enthalten.

Erfindungsgemäß ist, daß die Aminosäuren Glycin und/oder L-Alanin und/oder D-Alanin und/oder L-Valin und/oder D-Valin und/oder L-Threonin und/oder D-Threonin und/oder L-Asparinsäure und/oder D-Asparaginsäure und/oder L-Asparagin und/oder D-Asparagin und/oder L-Glutaminsäure und/oder D-Glutaminsäure und/oder L-Glutamin und/oder D-Glutamin und/oder L-Ornithin und/oder D-Ornithin und/oder L-Lysin und/oder D-Lysin und/oder 3-Aminopropansäure und/oder R-2-Aminobutansäure und S-2-Aminobutansäure und/oder 3-Aminobutansäure und/oder 4-Aminobutansäure und/oder R-2-Aminopentansäure und/oder S-2-Aminobutansäure und/oder 3-Aminopentansäure und/oder 4-Aminopentansäure und/oder 5-Aminopentansäure und/oder R-2-Aminohexansäure und/oder S-2-Aminohexansäure und/oder 3-Aminohexansäure und/oder 4-Aminohexansäure und/oder 5-Aminohexansäure und/oder 6-Aminohexansäure und/oder R-2-Aminoheptansäure und/oder S-2-Aminoheptansäure und/oder 3-Aminoheptansäure und/oder 4-Aminoheptansäure und/oder 5-Aminoheptansäure und/oder 6-Aminoheptansäure und/oder 7-Aminoheptansäure und/oder R-2-Aminooctansäure und/oder S-Aminooctansäure und/oder 3-Aminooctansäure und/oder 4-Aminooctansäure und/oder 5-Aminooctansäure und/oder 6-Aminooctansäure und/oder 7-Aminooctansäure und/oder 8-Aminooctansäure und/oder R-2-Aminononansäure und/oder S-2-Aminononansäure und/oder 3-Aminononansäure und/oder 4-Aminononansäure und/oder 5-Aminononansäure und/oder 6-Aminononansäure und/oder 7-Aminononansäure und/oder 8-Aminononansäure und/oder 9-Aminononansäure und/oder R-2-Aminodecansäure und/oder S-2-Aminodecansäure und/oder 3-Aminodecansäure und/oder 4-Aminodecansäure und/oder 5-Aminodecansäure und/oder 6-Aminodecansäure und/oder 7-Aminodecansäure und/oder 8-Aminodecansäure und/oder 9-Aminodecansäure und/oder 10-Aminodecansäure und/oder der 11-Aminoundecansäure und/oder L-Phenylalanin und/oder D-Phenylalanin und/oder L-Tyrosin und/oder D-Tyrosin und/oder L-Histidin und/oder D-Histidin und/oder L-Tryptophan und/oder D-Tryptophan als Bausteine der Oligoamide und Polyamide verwendet werden.

Erfindungsgemäß ist es von Vorteil, daß aliphatische Alkohle mit einer Anzahl von 12 bis 30 Kohlenstoffatomen als wasserfreie, organische Hilfskomponente verwendet werden.

Ferner ist erfindungsgemäß vorteilhaft, daß Fettsäuren mit einer Anzahl von 12 bis 30 Kohlenstoffatomen als wasserfreie, organische Hilfskomponente verwendet werden.

Erfindungsgemäß ist es auch vorteilhaft, daß Glycerintrifettsäureester, Glycerindifettsäureester und Glycerinmonofettsäureester als wasserfreie, organische Hilfskomponente bevorzugt sind, wobei die Fettsäurereste jeweils 14 bis 22 Kohlenstoffatome enthalten.

Erfindungsgemäß ist es von Vorteil, daß n-Alkane und/oder iso-Alkane mit 6 bis 30 Kohlenstoffatomen als wasserfreie, organische Hilfskomponente bevorzugt sind.

Erfindungsgemäß ist es vorteilhaft, daß Polyethylenglykol und/oder Polypropylenglykol mit Molmassen im Bereich von 200 bis 35000 als wasserfreie, organische Hilfskomponente bevorzugt sind.

Erfindungsgemäß ist es von Vorteil, daß Polyethylenoxid und/oder Polypropylenoxid mit Molmassen im Bereich von 35000 bis 1000000 als wasserfreie, organische Hilfskomponente bevorzugt sind.

Erfindungsgemäß ist es vorteilhaft, daß als wasserfreie, organische Hilfskomponente Gelatine, Kollagen, Cellulose, Carboxymethylcellulose, Methylcellulose, Ethylcellulose. Hydroxyethylcellulose, Propylcellulose, Hydroxypropylcellulose, Butylcellulose, Stärke, Carboxymethylstärke, Methylstärke, Ethylstärke, Hydroxyethylstärke, Propylstärke, Hydroxypropylstärke, Butylstärke, Chitin Carboxymethylchitin, Chitosan, Carboxymethylchitosan, Glykogen, Carboxymethylglykogen, Alginsäure, Alginsäuremethylether, Hyaluronsäure, Carboxymethylhyaluronsäure, Celluloseacetat, Cellulosepropionat, Cellulosebutyrat, Cellulosesulfat, Cellulosephosphat, Stärkeacetat, Stärkepropionat, Stärkebutyrat, Stärkesulfat, Stärkephosphat, oxidierte Cellulose, oxidierte Stärke, Pullulan, Araban, Xanthan und Guar Gum bevorzugt sind.

Erfindungsgemäß ist es vorteilhaft, daß als wasserfreie, organische Hilfskomponente Carnaubawachs, Bienenwachs, Benzoeharz, Kollophonium und Kopalharz bevorzugt sind.

Erfindungsgemäß ist es von Vorteil, daß als wasserfreie, organische Hilfskomponente Polyethylen, Polypropylen, Polybutadien, Polyisopren, Polychlorbutadien, Polymethylmethacrylat, Poly-2-hydroxyethylmethacrylat, Polymethylacrylat, Polystyrol, Polyvinylacetat, Polyvinylalkohol, Polyvinylchlorid, Polyvinylidenchlorid, Polyvinylfluorid, Polyvinylpyrrolidon, Polytetrafluorethylen, Polycarbonat, Polysulfon, Polysiloxan und Gemische dieser Polymere bevorzugt sind.

Erfindungsgemäß ist es vorteilhaft, daß als wasserfreie, organische Hilfskomponente Acrylsäurester, Acrylsäureamide, Methacrylsäurester, Methacrylsäureamide, Itaconsäureester, Maleinimide und deren Gemische bevorzugt sind.

Erfindungsgemäß ist es vorteilhaft, daß die wasserfreie, organische Hilfskomponente in festem und/oder in flüssigem Aggregatzustand vorliegt.

Auch erfindungsgemäß ist es von Vorteil, daß die Arylsulfate, Arylsulfonate, Arylsulfamate und Alkylarylsulfonate Bestandteile eines nichtvemetzten und/oder eines vernetzten Polymers sind, wobei Polymere aus der Gruppe der Polystyrole, der Polymethacrylate, Polyacrylate, Polyamide, der Polycarbonate und/oder deren Kopolymere und/oder deren Terpolymere bevorzugt sind.

Erfindungsgemäß ist es vorteilhaft, daß mindestens ein Antibiotikum aus den Gruppen der Penicillin-Antibiotika, der Cephalosporin-Antibiotika, der 4-Chinolon-Antibiotika und der Makrolid-Antibiotika oder mindestens ein Vertreter der Sulfonamid-Chemotherapeutika als biologisch aktive Hilfskomponente verwendet werden.

Erfindungsgemäß ist es vorteilhaft, daß ggf. Vertreter der Analgetika und/oder der Antiphlogistika als biologisch aktive Hilfskomponente verwendet werden.

Erfindungsgemäß ist weiterhin, daß die salzartige Komponente und die antibiotische Komponente in der wasserfreien, organischen Hilfskomponente suspendiert sind und eine injizierbare Suspension bilden.

Erfindungsgemäß ist, daß die Antibiotikum-/Antibiotika-Zubereitung, insbesondere injizierbare Suspension, zur Herstellung eines resorbierbaren Implantates und/oder eines nichtresorbierbaren Implantates verwendet wird.

Erfindungsgemäß ist ferner, daß die aus der Antibiotikum-/Antibiotika-Zubereitung hergestellten Formkörper, Granulate, Pulver, Schläuchen, Folien, Vliese und Fäden zur Herstellung eines resorbierbaren Implantates und/oder eines nichtresorbierbaren Implantates verwendet werden.

Erfindungsgemäß ist, daß die aus der Antibiotikum-/Antibiotika-Zubereitung hergestellten Formkörper, Granulate und Pulver plastisch verformbar und modellierbar sind.

Erfindungsgemäß ist ebenfalls, daß resorbierbare Implantate und nichtresorbierbare Implantante insbesondere in Form von Formkörpern, Granulaten, Pulvern, Schläuchen, Folien, Vliesen und Fäden mit der Antibiotikum-/Antibiotika-Zubereitung, insbesondere durch Pressen und/oder Tauchen und/oder Sprühen und/oder Kalandrieren und/oder Strangpressen und/oder Sintern und/oder Aufschmelzen, beschichtet werden.

Erfindungsgemäß ist, daß die Antibiotikum-/Antibiotika-Zubereitung als Beschichtung auf resorbierbare poröse Gläser, auf nichtresorbierbare poröse Gläser, auf resorbierbare poröse Glaskeramik, auf nichtresorbierbare poröse Glaskeramik, auf resorbierbare poröse Keramik und auf nichtresorbierbare poröse Keramik aufgebracht wird.

Schließlich ist es erfindungsgemäß, daß die Antibiotikum-/Antibiotika-Zubereitung als Beschichtung auf resorbierbare Kunststoffimplantate, auf nichtresorbierbare Kunststoffimplantate und auf Metallimplantate aufgebracht wird.

Der Gegenstand der vorliegenden Erfindung soll anhand der nachfolgenden Beispiele 1 - 2 näher erläutert werden.

### Herstellung der Antibiotikum-/Antibiotika-Zubereitungen

### Beispiel 1:

Es wird ein Gemisch aus 51 mg Gentamicinsulfat (700 U/mg, Fluka), 51 mg Natriumdodecylsulfat, 280 mg Camaubawachs, 1118 mg Calciumsulfat-Dihydrat (Fluka) und 1 ml Wasser hergestellt und nach dem Vermischen über Calciumchlorid getrocknet. Anschließend wird das Gemisch gemahlen. Jeweils 200 mg dieses Gemischs werden mit einer Presse bei einem Druck von 5 Tonnen innerhalb von zwei Minuten zu scheibenförmigen, stabilen Formkörpern, mit einem Durchmesser von 13 mm, gepreßt.

### Beispiel 2:

Es wird ein Gemisch aus 51 mg Gentamicinsulfat (700 U/mg, Fluka), 51 mg Natriumdodecylsulfat, 140 mg Bienenwachs, 1258 mg Calciumsulfat-Dihydrat (Fluka) und 1 ml Wasser hergestellt und nach dem Vermischen über Calciumchlorid getrocknet. Anschließend wird das Gemisch gemahlen. Jeweils 200 mg dieses Gemischs werden mit einer Presse bei einem Druck von 5 Tonnen innerhalb von zwei Minuten zu scheibenförmigen, stabilen Formkörpem, mit einem Durchmesser von 13 mm, gepreßt.

### Antibiotika-Freisetzungsversuche

Die in den Beispielen 1 und 2 hergestellten Formkörper wurden in physiologische Kochsalzlösung eingebracht und in dieser bei 37°C über einen Zeitraum von zwölf Tagen gelagert, um die retardierte Antibiotika-Freisetzung zu bestimmen. Die Probennahme erfolgte nach 1,3,6,9 und 12 Tagen Lagerungszeit. Die Antibiotika-Wertbestimmung wurde mit einem Agardiffusionstest unter Verwendung von Bacillus subtilis ATCC 6633 als Testkeim durchgeführt (Ergebnisse siehe **Tab.1**).

**Tab.1:**

| Kumulierte Gentamicin-Freisetzung aus Probekörpern der **Beispiele 1** und **2** in Abhängigkeit von der Lagerungszeit in physiologischer Kochsalzlösung bei 37°C. | | | | | |
|---|---|---|---|---|---|
| Beispiele | kumulierte Gentamicin-Freisetzung [Ma%] | | | | |
| | Lagerungszeit [d] | | | | |
| | 1 | 3 | 6 | 9 | 12 |
| 1 | 58 | 73 | 84 | 92 | 100 |
| 2 | 51 | 64 | 80 | 92 | 100 |

## Patentansprüche

1. Verfahren zur Herstellung einer Antibiotikum-/Antibiotika-Zubereitung, **dadurch gekennzeichnet, dass** Wasser, eine amphiphile Komponente eines Vertreters der Alkylsulfate, Arylsulfate, Alkylarylsulfate, Cycloalkylsulfate, Alkylcycloalkylsulfate, Alkylsulfamate, Cycloalkylsulfamate, Alkylcycloalkylsulfamate, Arylsulfamate, Alkylarylsulfamate, Alkylsulfonate, Fettsäure-2-sulfonate, Arylsulfonate, Alkylarylsulfonate, Cycloalkylsulfonate, Alkylcycloalkylsulfonate, Alkyldisulfate, Cycloalkyldisulfate, Alkyldisulfonate, Cycloalkyldisulfonate, Aryldisulfonate, Alkylaryldisulfonate, Aryltrisulfonate und Alkylaryltrisulfonate, mindestens eine antibiotische Komponente aus der Gruppe der Aminoglykosid-Antibiotika, der Lincosamid-Antibiotika und Tetracyclin-Antibiotika, eine anorganische Hilfskomponente **ggf.** eine organische Hilfskomponente, und ggf. mindestens eine biologisch aktive Hilfskomponente miteinander vermischt und ggf. zu Formkörpern und/oder Granulaten und/oder Pulvern und/oder Folien und/oder Vliesen und/oder Fäden geformt werden, **wobei als anorganische Hilfskomponente** Calciumhydrogenphosphat, Calciumhydrogenphosphat-Dihydraf, Hydroxylapatit, Fluorapatit, Calciumpolyphosphat, Tricalciumphosphat, Tefracalciumphosphat, Calciumsulfat, Calciumsulfat-Hemihydrat, Calciumsulfat-Dihydrat, Calciumlactat, Natriumhydrogencarbonat, Calciumcarbonat, Magnesiumcarbonat, Calciumhydroxid, Magnesiumhydroxid, Magnesiumoxid **oder** Gemische dieser Stoffe in Form grobdisperser (0,5 bis 2 mm) und/oder hochdisperser Pulver **oder** resorbierbare Gläser, nichtresorbierbare Gläser, resorbierbare Glaskeramik, nichtresorbierbare Glaskeramik, resorbierbare Keramik und nichtresorbierbare Keramik verwendet werden.

2. Verfahren zur Herstellung einer Antibiotikum-/Antibiotika-Zubereitung nach Anspruch 1, **dadurch gekennzeichnet, dass** das Formen mittels Pressen und/oder Strangpressen und/oder Mahlen und/oder Kalandrieren und/oder Gießen und/oder Spinnen und/oder Sintern bewerkstelligt wird.

3. Verfahren zur Herstellung einer Antibiotikum-/Antibiotika-Zubereitung nach einem der Ansprüche 1 bis 2, **dadurch gekennzeichnet, dass** die antibiotische Komponente mindestens eine Aminogruppe enthält.

4. Verfahren zu Herstellung einer Antibiotikum-/Antibiotika-Zubereitung nach einem der Ansprüche 1 bis 3, **dadurch gekennzeichnet, dass** die antibiotische Komponente in der protonierten Salzform vorliegt, wobei Chlorid-lonen, Bromid-Ionen, Hydrogensulfat-lonen, Sulfat-Ionen, Dihydrogenphosphat-lonen, Hydrogenphosphat-lonen, Phosphat-Ionen, Acetat-Ionen, Succinat-lonen und Lactat-lonen als Gegenionen verwendet werden.

5. Verfahren zur Herstellung einer Antibiotikum-/Antibiotika-Zubereitung nach einem der Ansprüche 1 bis 4, **dadurch gekennzeichnet, dass** das Verhältnis der Stoffmenge der amphiphilen Komponente zu der Stoffmenge der antibiotischen Komponente im Bereich von 0,01 bis 10 liegt.

6. Verfahren zur Herstellung einer Antibiotikum-/Antibiotika-Zubereitung nach einem der Ansprüche 1 bis 5, **dadurch gekennzeichnet, dass** die organische Hilfskomponente hydrolytisch spaltbare Carbonsäureesterbindungen und/oder hydrolytisch spaltbare Carbonsäureamidbindungen und/oder hydrolytisch spaltbare Carbonsäureanhydridbindungen und/oder hydrolytisch spaltbare Phosphorsäuresterbindungen und/oder hydrolytisch spaltbare Phosphorsäureamidbindungen und/oder enzymatisch spaltbare Carbonsäureesterbindungen und/oder enzymatisch spaltbare Carbonsäureamidbindungen und/oder enzymatisch spaltbare Carbonsäureanhydridbindungen und/oder enzymatisch spaltbare Phosphorsäuresterbindungen und/oder enzymatisch spaltbare Phosphorsäureamidbindungen aufweist.

7. Verfahren zur Herstellung einer Antibiotikum-/Antibiotika-Zubereitung nach einem der Ansprüche 1 bis 6, **dadurch gekennzeichnet, dass** die organische Hilfskomponente in festem und/oder in flüssigem Aggregatzustand vorliegt.

8. Verfahren zur Herstellung einer Antibiotikum-/Antibiotika-Zubereitung nach einem der Ansprüche 1 bis 7, **dadurch gekennzeichnet, dass** die Arylsulfate, Arylsulfonate, Arylsulfamate und Alkylarylsulfonate Bestandteile eines nichtvernetzten und/oder eines vernetzten Polymers sind, wobei Polymere aus der Gruppe der Polystyrole, der Polymethacrylate, Polyacrylate, Polyamide, der Polycarbonate und/oder deren Kopolymere und/oder deren Terpolymere verwendet werden.

9. Antibiotikum-/Antibiotika-Zubereitung, hergestellt nach einem Verfahren nach einem der Ansprüche 1 bis 8.

10. Antibiotikum-/Antibiotika-Zubereitung, hergestellt nach einem Verfahren nach einem der Ansprüche 1 bis 8, **dadurch gekennzeichnet, dass** die Antibiotikum-/Antibiotika-Zubereitung eine injizierbare Suspension ist.

11. Verwendung einer Antibiotikum-/Antibiotika-Zubereitung, hergestellt nach einem der Ansprüche 1 bis 8; zur Herstellung eines resorbierbaren oder eines nichtresorbierbaren Implantates.

12. Verwendung von aus einer Antibiotikum-/Antibiotika-Zubereitung, hergestellt nach einem der Ansprüche 1 bis 8, hergestellten Formkörpem, Granulaten, Pulvern, Folien, Fäden und Vliesen zur Herstellung eines resorbierbaren Implantates und/oder eines nichtresorbierbaren Implantates.

13. Verwendung einer Antibiotikum-/Antibiotika-Zubereitung zur Herstellung eines Arzneimittels, hergestellt nach einem der Ansprüche 1 bis 8, **dadurch gekennzeichnet, dass** aus der Antibiotikum-/Antibiotika-Zubereitung hergestellte Formkörper, Granulate und Pulver plastisch verformbar und modellierbar sind.

14. Verwendung einer Antibiotikum-/Antibiotika-Zubereitung, hergestellt nach einem der Ansprüche 1 bis 8 zur Beschichtung von resorbierbaren Implantaten oder nichtresorbierbaren Implantanten.

15. Verwendung einer Antiblotikum-/Antibiotika-Zubereitung zur Herstellung eines Arzneimittels, hergestellt nach einem der Ansprüche 1 bis 8, **dadurch gekennzeichnet, dass** die Antibiotikum-/Antibiotika-Zubereitung als Beschichtung auf resorbierbare poröse Gläser, auf nichtresorbierbare poröse Gläser, auf resorbierbare poröse Glaskeramik, auf nichtresorbierbare poröse Glaskeramik, auf resorbierbare poröse Keramik und auf nichtresorbierbare poröse Keramik aufgebracht wird.

16. Verwendung einer Antibiotikum-/Antibiotika-Zubereitung zur Herstellung eines Arzneimittels, hergestellt nach einem der Ansprüche 1 bis 8, **dadurch gekennzeichnet, dass** die Antibiotikum-/Antibiotika-Zubereitung als Beschichtung auf resorbierbare Kunststoffimplantate, auf nichtresorbierbare Kunststoffimplantate und auf Metallimplantate aufgebracht wird.

## Claims

1. Process for the preparation of a formulation comprising an antibiotic/antibiotics, **characterized in that** water, an amphiphilic component of a member of the alkylsulphates, arylsulphates, alkylarylsulphates, cycloalkylsulphates, alkylcycloalkylsulphates, alkylsulphamates, cycloalkylsulphamates, alkylcycloalkylsulphamates, arylsulphamates, alkylarylsulphamates, alkylsulphonates, fatty acid-2-sulphonates, arylsulphonates, alkylarylsulphonates, cycloalkylsulphonates, alkylcycloalkylsulphonates, alkyldisulphates, cycloalkyldisulphates, alkyldisulphonates, cycloalkyldisulphonates, aryldisulphonates, alkylaryldisulphonates, aryltrisulphonates and the alkylaryltrisulphonates, at least one antibiotic component from the group consisting of the aminoglycoside antibiotics, the lincosamide antibiotics and tetracycline antibiotics, an inorganic auxiliary component, optionally an organic auxiliary component and optionally at least one biologically active auxiliary component are mixed with one another and optionally shaped into mouldings and/or granules and/or powders and/or films and/or nonwovens and/or filaments, wherein calcium hydrogen phosphate, calcium hydrogen phosphate dihydrate, hydroxyapatite, fluoroapatite, calcium polyphosphate, tricalcium phosphate, tetracalcium phosphate, calcium sulphate, calcium sulphate hemihydrate, calcium sulphate dihydrate, calcium lactate, sodium bicarbonate, calcium carbonate, magnesium carbonate, calcium hydroxide, magnesium hydroxide, magnesium oxide or mixtures of these substances in the form of coarsely disperse (0.5 to 2 mm) and/or highly disperse powders or absorbable glasses, nonabsorbable glasses, absorbable glass ceramic, nonabsorbable glass ceramic, absorbable glass ceramic and nonabsorbable ceramic is used as the inorganic auxiliary component.

2. Process for the preparation of a formulation comprising an antibiotic/antibiotics according to Claim 1, **characterized in that** the shaping is effected by means of compression and/or extrusion and/or milling and/or calendering and/or casting and/or spinning and/or sintering.

3. Process for the preparation of a formulation comprising an antibiotic/antibiotics according to either of Claims 1 and 2, **characterized in that** the antibiotic component contains at least one amino group.

4. Process for the preparation of a formulation comprising an antibiotic/antibiotics according to any of Claims 1 to 3, **characterized in that** the antibiotic component is present in the protonated salt form, chloride ions, bromide ions, hydrogen sulphate ions, sulphate ions, dihydrogen phosphate ions, hydrogen phosphate ions, phosphate ions, acetate ions, succinate ions and lactate ions being used as opposite ions.

5. Process for the preparation of a formulation comprising an antibiotic/antibiotics according to any of Claims 1 to 4, **characterized in that** the ratio of the amount of the amphiphilic component to the amount of the antibiotic component is in the range of 0.01 to 10.

6. Process for the preparation of a formulation comprising an antibiotic/antibiotics according to any of Claims 1 to 5, **characterized in that** the organic auxiliary component has hydrolytically cleavable carboxylic ester bonds and/or hydrolytically cleavable carboxamide bonds and/or hydrolytically cleavable carboxylic anhydride bonds and/or hydrolytically cleavable phosphoric ester bonds and/or hydrolytically cleavable phosphoramide bonds and/or enzymatically cleavable carboxylic ester bonds and/or enzymatically cleavable carboxamide bonds and/or enzymatically cleavable carboxylic anhydride bonds and/or enzymatically cleavable phosphoric ester bonds and/or enzymatically cleavable phosphoramide bonds.

7. Process for the preparation of a formulation comprising an antibiotic/antibiotics according to any of Claims 1 to 6, **characterized in that** the organic component is present in the solid and/or in the liquid state of aggregation.

8. Process for the preparation of a formulation comprising an antibiotic/antibiotics according to any of Claims 1 to 7, **characterized in that** the arylsulphates, arylsulphonates, arylsulphamates and alkylarylsulphonates are components of an uncrosslinked and/or of a crosslinked polymer, polymers from the group consisting of the polystyrenes, of the polymethacrylates, polyacrylates, polyamides, of the polycarbonates and/or of the copolymers thereof and/or of the terpolymers thereof being used.

9. Formulation comprising an antibiotic/antibiotics, prepared by a process acceding to any of Claims 1 to 8.

10. Formulation comprising an antibiotic/antibiotics, prepared by a process according to any of Claims 1 to 8, **characterized in that** the formulation comprising an antibiotic/antibiotics is an injectable suspension.

11. Use of a formulation comprising an antibiotic/antibiotics, prepared according to any of Claims 1 to 8, for the production of an absorbable or of a nonabsorbable implant.

12. Use of mouldings, granules, powders, films, filaments and nonwovens produced from a formulation comprising an antibiotic/antibiotics, prepared according to any of Claims 1 to 8, for the production of an absorbable implant and/or of a nonabsorbable implant.

13. Use of a formulation comprising an antibiotic/antibiotics for the preparation of a drug, prepared according to any of Claims 1 to 8, **characterized in that** mouldings, granules and powders produced from the formulation comprising an antibiotic/antibiotics are plastically deformable and capable of being modelled.

14. Use of a formulation comprising an antibiotic/antibiotics, prepared according to any of Claims 1 to 8, for the coating of absorbable implants or nonabsorbable implants.

15. Use of a formulation comprising an antibiotic/antibiotics for the preparation of a drug, prepared according to any of Claims 1 to 8, **characterized in that** the formulation comprising an antibiotic/antibiotics is applied in the form of a coating to absorbable porous glasses, to nonabsorbable porous glasses, to absorbable porous glass ceramic, to nonabsorbable porous glass ceramic, to absorbable porous ceramic and to nonabsorbable porous ceramic.

16. Use of a formulation comprising an antibiotic/antibiotics for the preparation of a drug, prepared according to any of Claims 1 to 8, **characterized in that** the formulation comprising an antibiotic/antibiotics is applied in the form of a coating to absorbable plastic implants, to nonabsorbable plastic implants and to metal implants.

## Revendications

1. Procédé de production d'une préparation d'antibiotique/antibiotiques **caractérisé en ce que** de l'eau, un composant amphiphile d'un représentant des alkylsulfates, arylsulfates, alkylarylsulfates, cycloalkylsulfates, alkylcycloalkylsulfates, alkylsulfamates, cycloalkylsulfamates, alkylcycloalkylsulfamates, arylsulfamates, alkylarylsulfamates, alkylsulfonates, acide gras-2-sulfonates, arylsulfonates, alkylarylsulfonates, cycloalkylsulfonates, alkylcycloalkylsulfonates, alkyldisulfates, cycloalkyldisulfates, alkyldisulfonates, cycloalkyldisulfonates, aryldisulfonates, alkylaryldisulfonates, aryltrisulfonates et alkylaryltrisulfonates, au moins un composant antibiotique du groupe des antibiotiques de type aminoglycoside, des antibiotiques de type lincosamide et des antibiotiques de type tétracycline, éventuellement un composant auxiliaire organique, un composant auxiliaire inorganique et éventuellement au moins un composant auxiliaire biologiquement actif sont mélangés entre eux et sont éventuellement mis sous forme de corps mis en forme et/ou de granulés et/ou de poudres et/ou de feuilles et/ou de nappes et/ou de fils, où l' hydrogénophosphate de calcium, l'hydrogénophosphate de calcium dihydraté, l'hydroxylapatite, la fluoroapatite, le polyphosphate de calcium, le phosphate de tricalcium, le phosphate de tétracalcium, le sulfate de calcium, le sulfate de calcium hémihydraté, le sulfate de calcium dihydraté, le lactate de calcium, l'hydrogénocarbonate de sodium, le carbonate de calcium, le carbonate de magnésium, l'hydroxyde de calcium, l'hydroxyde de magnésium, l'oxyde de magnésium ou des mélanges de ces substances sous forme de poudre dispersée grossièrement (0,5 à 2 mm) et/ou hautement dispersée ou de verres résorbables, de verres non résorbables, de vitrocéramiques résorbables, de vitrocéramiques non résorbables, de céramiques résorbables et de céramiques non résorbables sont utilisés comme composant auxiliaire inorganique.

2. Procédé de production d'une préparation d'antibiotique/antibiotiques selon la revendication 1 **caractérisé en ce que** la mise en forme est réalisée par compression et/ou extrusion et/ou broyage et/ou calandrage et/ou coulée et/ou filage et/ou frittage.

3. Procédé de production d'une préparation d'antibiotique/antibiotiques selon l'une des revendications 1 à 2 **caractérisé en ce que** le composant antibiotique contient au moins un groupe amino.

4. Procédé de production d'une préparation d'antibiotique/antibiotiques selon l'une des revendications 1 à 3 **caractérisé en ce que** le composant antibiotique est présent sous forme saline protonée, où des ions chlorure, des ions bromure, des ions hydrogénosulfate, des ions sulfate, des ions dihydrogénophosphate, des ions hydrogénosphosphate, des ions phosphate, des ions acétate, des ions succinate et des ions lactate sont utilisés comme contre-ions.

5. Procédé de production d'une préparation d'antibiotique/antibiotiques selon l'une des revendications 1 à 4 **caractérisé en ce que** le rapport de la quantité de substance du composant amphiphile à la quantité de substance du composant antibiotique est situé dans le domaine de 0,01 à 10.

6. Procédé de production d'une préparation d'antibiotique/antibiotiques selon l'une des revendications 1 à 5 **caractérisé en ce que** le composant auxiliaire organique comporte des liaisons ester d'acide carboxylique clivables par hydrolyse et/ou des liaisons amide d'acide carboxylique clivables par l'hydrolyse et/ou des liaisons anhydride d'acide carboxylique clivables par hydrolyse et/ou des liaisons ester d'acide phosphorique clivables par hydrolyse et/ou des liaisons amide d'acide phosphorique clivables par hydrolyse et/ou des liaisons ester d'acide carboxylique clivables par voie enzymatique et/ou des liaisons amide d'acide carboxylique clivables par voie enzymatique et/ou des liaisons anhydride d'acide carboxylique clivables par voie enzymatique et/ou des liaisons ester d'acide phosphorique clivables par voie enzymatique et/ou des liaisons amide d'acide phosphorique clivables par voie enzymatique.

7. Procédé de production d'une préparation d'antibiotique/antibiotiques selon l'une des revendications 1 à 6 **caractérisé en ce que** le composant auxiliaire organique est dans un état physique solide et/ou liquide.

8. Procédé de production d'une préparation d'antibiotique/antibiotiques selon l'une des revendications 1 à 7 **caractérisé en ce que** les arylsulfates, arylsulfonates, arylsulfamates et alkylarylsulfonates sont des constituants d'un polymère non réticulé et/ou réticulé, où des polymères du groupe des polystyrènes, des polyméthacrylates, des polyacrylates, des polyamides, des polycarbonates et/ou de leurs copolymères et/ou de leurs terpolymères sont utilisés.

9. Préparation d'antibiotique/antibiotiques produite selon un procédé selon l'une des revendications 1 à 8.

10. Préparation d'antibiotique/antibiotiques produite selon un procédé selon l'une des revendications 1 à 8 **caractérisée en ce que** la préparation d'antibiotique/antibiotiques est une suspension injectable.

11. Utilisation d'une préparation d'antibiotique/antibiotiques produite selon l'une des revendications 1 à 8 pour la production d'un implant résorbable ou d'un implant non résorbable.

12. Utilisation de corps mis en forme, de granulés, de poudres, de feuilles, de fils et de nappes produits à partir d'une préparation d'antibiotique/antibiotiques produite selon l'une des revendications 1 à 8 pour la production d'un implant résorbable et/ou d'un implant non résorbable.

13. Utilisation, pour la production d'un médicament, d'une préparation d'antibiotique/antibiotiques produite selon l'une des revendications 1 à 8 **caractérisée en ce que** des corps mis en forme, des granulés et des poudres produits à partir de la préparation d'antibiotique/antibiotiques peuvent être déformés et façonnés plastiquement.

14. Utilisation d'une préparation d'antibiotique/antibiotiques produite selon l'une des revendications 1 à 8 pour le revêtement d'implants résorbables ou d'implants non résorbables.

15. Utilisation, pour la production d'un médicament, d'une préparation d'antibiotique/antibiotiques produite selon l'une des revendications 1 à 8 **caractérisée en ce que** la préparation d'antibiotique/antibiotiques est appliquée comme revêtement sur des verres poreux résorbables, sur des verres poreux non résorbables, sur des vitrocéramiques poreuses résorbables, sur des vitrocéramiques poreuses non résorbables, sur des céramiques poreuses résorbables et sur des céramiques poreuses non résorbables.

16. Utilisation, pour la production d'un médicament, d'une préparation d'antibiotique/antibiotiques produite selon l'une des revendications 1 à 8 **caractérisée en ce que** la préparation d'antibiotique/antibiotiques est appliquée comme revêtement sur des implants en matière plastique résorbables, sur des implants en matière plastique non résorbables et sur des implants métalliques.
